# EUROPEAN PATENT SPECIFICATION

(11) **EP 4 436 519 B1**
(45) Date of publication and mention of the grant of the patent: **25.02.2026**
(21) Application number: 22838989.6
(22) Date of filing: 24.11.2022
(51) Int. Cl.: A61B 17/064, A61F 2/16

(54) **MEDICAL DEVICE FOR FIXING INTRAOCULAR LENSES**
MEDIZINISCHE VORRICHTUNG ZUR FIXIERUNG VON INTRAOKULARLINSEN
DISPOSITIF MÉDICAL POUR FIXER DES LENTILLES INTRAOCULAIRES

(30) Priority: 24.11.2021 IT 202100029747
(43) Date of publication of application: 02.10.2024
(73) Proprietor: Rossi, Tommaso, 00165 Roma (IT); Malvasi, Carlo, 00137 Roma (IT); Silva, Paolo, 26100 Cremona (IT)
(72) Inventor: Rossi, Tommaso, 00165 Roma (IT); Malvasi, Carlo, 00137 Roma (IT); Silva, Paolo, 26100 Cremona (IT)
(74) Representative: Laghi, Alberto
(86) International application number: PCT/IT2022/050302
(87) International publication number: WO 2023/095180

(56) References cited:
- WO-A1-2012/135530
- WO-A1-2014/035862
- WO-A2-2011/116228
- US-A1- 2015 025 540
- US-A1- 2021 161 652
- OLSON JEFFREY L ET AL: "Intraocular lens fixation with a 30-gauge injectable shape-memory alloy clip in a porcine eye", JOURNAL CATARACT AND REFRACTIVE SURGERY, SURGERY, FAIRFAX, VA, US, vol. 38, no. 6, 6 April 2012 (2012-04-06), pages 1105 - 1106, XP028923147, ISSN: 0886-3350, DOI: 10.1016/J.JCRS.2012.04.016

## Description

### Field of the Invention

The present invention relates to a medical device for fixing intraocular lenses to the scleral wall during ophthalmic surgery. More specifically, the invention relates to a device which performs the fixation and/or stabilization, during an ophthalmic surgery operation, of a previously or contextually implanted intraocular lens (IOL), adapting to use in combination with any type of intraocular lens.

### Background of the invention

Cataract, a widespread eye disease all over the world, is the result of a loss of transparency of the crystalline lens, the optical organ responsible for keeping the visual image in focus on the retina. The lens is a bi-convex lens of transparent protein material, with a diameter of about 9 mm, enclosed in a membrane called the capsular sac, suspended inside the eye, between the iris and the vitreous body, and kept in central position in correspondence with the visual axis by an anchoring system to the ocular wall. This system consists of a myriad of thin filaments, called Zinn's zonula, which with radial symmetry extend from the ciliary body, the structure that regulates the accommodative process with the ciliary muscle, to the equator of the capsular bag.

Cataract surgery consists in the removal of the opacified lens and its replacement with an artificial lens. It represents the most frequent surgery ever in every medical specialty, with over 20 million operations per year worldwide, 550,000 in Italy and about 7 million in Europe. The artificial lens or intraocular lens (IOL) has been routinely implanted since the end of the 70s in all cataract surgeries, in order to obtain a complete rehabilitation of patients, who often return to 10/10 vision in the absence of concomitant diseases.

The elective site for positioning the artificial lens, which completely restores the preoperative anatomy, is inside the capsular bag, but depending on the situation, it can also be positioned in different anatomical sites. In fact, depending on the surgical technique adopted or specific patient problems, it may be necessary to implant the IOL in alternative locations, such as the ciliary sulcus (the space between the posterior face of the base of the iris and the anterior face of the ciliary body, in the posterior chamber) or, much less often, in the anterior chamber (in front of the iris).

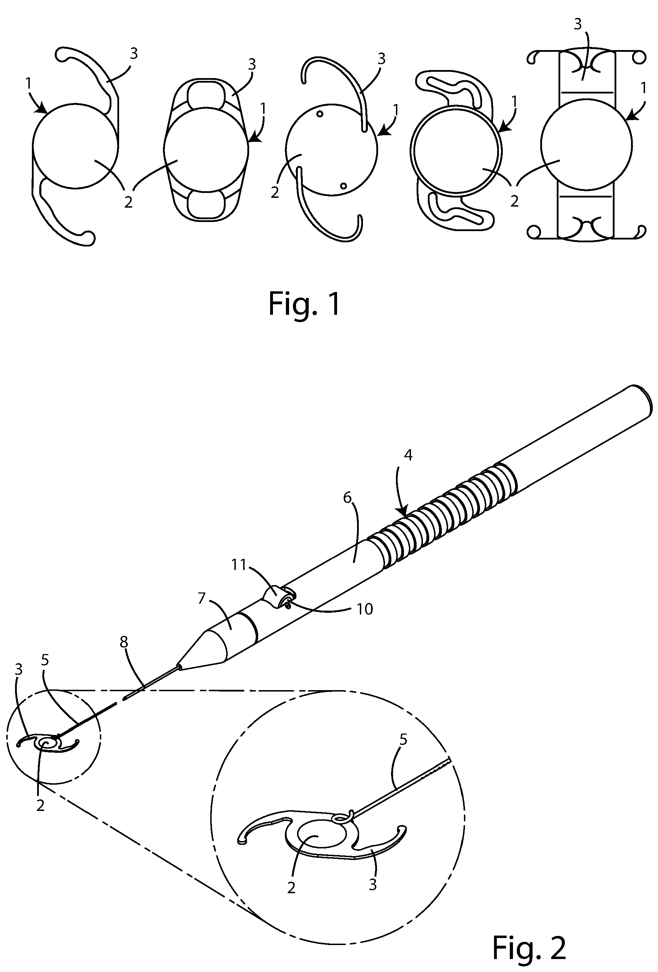

The support system of an intraocular lens inside the eye (more often, in the capsular bag) is commonly made up of two opposing elastic appendages or struts, called haptics, which, leaning inside the capsular bag or wall of the anterior segment of the eye, symmetrically and concentrically repel the centrally located intraocular lens. By way of illustration, **Figure 1** of the accompanying drawings shows some examples of intraocular lens (1), each characterized by a central disc (2), called optical plate, and by the already mentioned elastic appendages (3), which allow it to be correct positioning within the implant site.

The intraocular lens, implanted in place of the natural opacified crystalline, allows obtaining a significant rehabilitation of the visual capacity in cases where cataract surgery has become necessary. However, the structure which supports the capsular bag (the Zinn zonula) and the capsule itself, over time, can alter or become damaged, and can lose its ability to support the IOL in its correct and central position, due to aging or to very frequent pathologies such as *pseudoesfoliatio lentis,* high myopia, or even due to blunt trauma. In all cases in which the artificial lens is decentralized or inclined with respect to its optimal position, surgery becomes necessary in order to fix it in an adequate position, or to replace it with one of a different design, specifically conceived to be fixed with a series of techniques surgical procedures, collectively referred to as "scleral fixation".

The scleral fixation techniques of the artificial crystalline lens involve maneuvers aimed at anchoring the haptics of the IOL or, much less frequently, its optical plate, to the sclera, to the *sulcus ciliare* or to the iris by means of suitable sutures, or by creating suitably made scleral pockets to accommodate the haptics, portions thereof, or of the optical plate even in the absence of sutures (Yamane technique). These techniques present a very large number of different variants, firstly because each of them has been conceived for specific IOL geometries and for particular cases. Other techniques provide for the replacement of the IOL with specifically designed lenses having hooks to accommodate sutures for fixation, or lenses provided with hooks designed to be anchored to the sclera in sutureless techniques.

By way of example, the international patent application publ. No. WO2014/138615 (in the name of Optic Logic LLC) discloses a device aimed at engaging the haptics of a displaced intraocular lens (in this case a lens positioned in the *sulcus ciliare*) by means of a variety of alternative clip elements. The device is designed to pull the haptics once hooked, in order to bring the lens back to the correct position and finally anchor it with special sutures to the scleral tissue.

A further example of a device proposed (also) for the scleral fixation of intraocular lenses is represented by the published patent application US 2021/0161652 (in the name of Barnett, assigned to the Duke University of Durham, NC). Said document discloses a fixation device based on a shape memory element, which can be implanted by means of a cannula or other suitable injecting surgical device. According to the disclosure, the device comprises a shape memory main body which can be compressed, lengthened or shortened inside the cannula, and which, once expelled, regains its memorized extended shape. The collapsible main body includes terminal hooks by which the shape memory element can be fixed to the scleral wall once expanded.

The expression shape memory alloys (SMA) refers to a particular set of metal alloys characterized by two properties:
1. shape memory effect (SME), i.e. the ability to recover, following an appropriate change in environmental conditions, generally the temperature, a pre-set geometric shape obtained through repeated heat treatments (training);
2. superelasticity (SE), i.e. the ability to withstand, within a given temperature range, large deformations (up to 10-15%) with respect to the initial configuration without showing permanent effects.

Shape memory metal alloys have been created and studied since the 1930s, but it was in the 1960s that these materials achieved commercial diffusion, with the development of the first nickel-titanium alloys, marketed under the trade name Nitinol. These alloys are widely used, in particular, in the biomedical field for sutures, stents, endovascular prostheses, surgical instruments and other similar applications, both in view of the aforementioned characteristics, which have allowed the development of minimally invasive surgery techniques, and in view of their excellent biocompatibility, which makes these materials also suitable for implantology. In addition to thermally activated shape memory alloys, for example, ferromagnetic shape memory alloys have been discovered and developed, which temporarily change their shape under the action of magnetic fields. In general, shape memory is a property obtained thanks to the coexistence in the material of two separate phases with different properties, which are capable of reacting to an external stimulus in different ways. Theoretically, therefore, any material with these characteristics can have shape memory behaviour, and this is what has allowed polymers to become popular in this field as well.

Shape memory polymers (SMP) are defined as polymers having the ability to return from a deformed state (temporary shape) to their original shape (permanent shape) through an external stimulus (activation). Polymers are able to show an enormous versatility in terms of activation methods, to the point that materials activated by heat, electric current, application of forces, light radiation and hydration have been developed. In addition to the different types of activation, the versatility of the polymers is also due to the fact that shape memory can be obtained both on thermoplastic and thermosetting polymers, using cross-linked polymers, block copolymers or polymer blends.

With reference to medical devices which exploit the properties of shape memory alloys, and in particular of Nitinol, to achieve the fixation of a dislocated intraocular lens, US patent 9474596 (in the name of Khalid Sabti), discloses a device equipped with an elongated hollow handpiece, connected in its distal part to a suction pump, which has a tubular Nitinol neck in its proximal part, intended to be introduced into the operating field in contact with the IOL to be repositioned. While the suction pump is intended to recover a dislocated IOL by suction, the tubular Nitinol neck, which is fixed to the handpiece, can vary its conformation between elongated and spiral (two-way effect, obtainable in the material by means of appropriate training cycles) in order to impart a rotational movement to the aspirated IOL. The rotational movement would allow the rotational repositioning of the lens inside the eye.

All the scleral fixation techniques mentioned are surgical techniques characterized by a high level of complexity and expensive in terms of time and materials, they require the intervention of expert surgeons and do not exclude the occurrence of damage, even permanent, to the patient's visual system.

Furthermore, it should be noted that none of the hitherto known fixation techniques can be used with all types of lenses and in all types of lens dislocations; moreover, no intraocular lens normally implanted in the capsular bag at the end of an operation without complications is properly suited to scleral fixation techniques except in the face of extremely particular and risky surgical manoeuvers. This is why it is often necessary to explant the present IOL and to implant an IOL with a geometry suitable for the desired fixation. In such cases, the patient is exposed to the trauma of undergoing the explantation of the existing lens to implant one with a geometry more favourable to the new positioning and to the different fixation method. Given that the removal of the IOL is a complex surgical manoeuver, not free from risks of complications and damage to the surrounding tissues, mainly the cornea, it is desirable to avoid it whenever repositioning and acceptable fixation of the present IOL is possible.

From the above considerations, it is clear that the techniques currently adopted for the repositioning and scleral fixation of a dislocated artificial lens, in addition to being extremely demanding and complex, are not applicable indifferently to any type of intraocular lens, and also involve a considerable risk of damage to the ocular structures, and therefore to the patient's vision.

### Summary of the invention

The object of the present invention is, to provide a device capable of fixing, centering and securing any type of previously or simultaneously implanted intraocular lens regardless of the configuration of the latter, in order to avoid removal of the existing IOL in favour of a specific lens for fixation, thus allowing the repositioning of the existing lens in a predetermined and safe manner, so as to reduce possible damage to the visual apparatus.

According to the present invention, it has been found that by using the superelastic properties of shape memory materials for the production of specifically preconfigured elements it is possible to engage in a pre-defined manner any intraocular lens at the edges of its optical plate and anchor it to the scleral wall, in order to standardize the scleral fixation technique. The specific configurations spontaneously assumed by the shape memory elements of the invention according to whether they are in the external environmental conditions or within the ocular tissues also allow reducing the level of complexity of the operation. Also, the removal of the present lens in favour of a specific lens is avoided, thus guaranteeing the best visual capacity and the safeguarding of the patient's ocular tissues, with a considerable saving of surgical time and invasiveness.

The present invention, therefore, comprises a surgical device which, by allowing an easy access to the operating field, allows any intraocular lens to be mechanically engaged by a suitably pre-set three-dimensional shape memory element. The latter is expelled from the surgical device when it is in a substantially rectilinear conformation, suitable for expulsion, and returns to its pre-set conformation when it is positioned inside the eye after expulsion, engaging the displaced IOL and stabilizing its position on the visual axis of the patient.

By means of the surgical device studied according to the invention, once the intraocular lens, dislocated for any reason, has been grasped with an intraocular forceps or in any case repositioned in the capsular sac, in the ciliary sulcus in the posterior chamber, or in anterior chamber, an injector handpiece allows the surgeon to inject a substantially rectilinear shape memory element, whose pre-set configuration, once recovered, is such as to make it interact mechanically with the displaced intraocular lens, so as to stabilize the position of the lens and to allow the surgeon to fix it stably to the scleral wall, creating a sort of "anchoring strut".

By means of the three-dimensional shape memory element according to the invention, which is stably implanted into the eye to grasp the IOL in proximity to the margin of the optical plate in at least two diametrically opposed thereof, a minimally invasive and extremely simple technique is realised. Such technique, which is applicable to any type of IOL, allows permanently avoiding new dislocations of the same, and reduces the risk of damage and complications to the patient's visual apparatus.

### Brief description of the figures

The specific characteristics of the invention, as well as the advantages thereof, will become more evident with reference to some of its preferred embodiments illustrated in the accompanying figures, in which:
**Figure 1****,** already discussed above, shows some examples of intraocular lenses of the current technique, to illustrate the variety of types of IOLs for which the device according to the present invention can be used for scleral fixation;
**Figure 2** shows a perspective view with an enlarged detail of a first embodiment of the medical device for scleral fixation according to the invention, represented at the moment in which it engages an intraocular lens;
**Figure 3** shows a longitudinal sectional view of the injector handpiece of the medical device of Figure 2, with a wire of shape memory material inserted inside it to be injected to perform scleral fixation;
**Figure 4** shows a front view of an intraocular lens engaged by two shape memory elements expelled from the medical device of Figure 2 in two different moments of the operation performed with the use of the device;
**Figure 5** shows a schematic front view of the same elements of Figure 4 seen in the eyeball in which they are implanted;
**Figures 6A, 6B** and **6C** show, respectively, a rear view, a front view and a side elevation view of an intraocular lens engaged by two shape memory elements according to a second embodiment of the invention, ejected from the injector handpiece of the medical device of Figure 2 each at a first moment of the operation performed with the use of the device; and
**Figures 7A, 7B** and **7C** show, respectively, a rear view, a front view and a side elevation view of the same intraocular lens engaged in Figures 6A, 6B and 6C at a moment of the operation subsequent to those schematically shown in the previous three figures, i.e. after insertion of the fixing means and achievement of the memorized configuration, conforming to the scleral profile.

### Detailed description of the invention

Therefore, the present invention specifically concerns a system for scleral fixation and stabilization of a previously or simultaneously implanted intraocular lens, said intraocular lens having a central optical plate and two or more peripheral elastic haptics, which system comprises:
- a medical device consisting of an injector handpiece (4) with an internal longitudinal cavity ending with a hollow tubular proximal end (7, 8) open towards the outside, suitable for introducing into an operating field of ophthalmic surgery a three-dimensional shape memory implantable element (5) housed therein, having a substantially rectilinear configuration at rest;
- one or more further three-dimensional shape memory implantable elements (5) having a substantially rectilinear configuration at rest, each to be inserted in said injector handpiece (4) in place of the previously injected one;

wherein said three-dimensional shape memory implantable elements (5) have a memorized configuration capable of engaging and retaining, with a first end (12) in the shape of a an intraocular lens (1) at the margins of the optical plate (2) of the same, and with the second end (13) anchored to the scleral wall,
and wherein said three-dimensional shape memory implantable elements (5) autonomously assume the memorized configuration when exposed to a predetermined environmental condition corresponding to the conditions inside the eye.

According to a preferred embodiment of the invention, said predetermined environmental condition is the temperature, and the shape memory elements assume the memorized configuration when brought to the internal temperature of the eye.

More generally, the three-dimensional shape memory implantable element is treated, in the construction stage, in such a way as to be able to autonomously assume, in correspondence with a predetermined environmental condition determined by the values assumed by one or more intensive physical quantities, for example the temperature, a not limitedly filiform profile, lying on multiple geometric planes and capable of engaging and retaining any intraocular lens in the patient's eye.

In its configuration at rest, in environmental conditions different from those inside the patient's eye, the shape memory element of the surgical device according to the invention has a curvilinear filiform shape lying in a single plane, or rather rectilinear, and therefore it can be housed inside the cannula of the injector handpiece. Under the action performed by the mechanism inside the handpiece, the shape memory element enters the operating site, and due to the environmental conditions present in the operating field, and thanks to its own characteristics, including superelasticity, it autonomously changes its configuration, reacquiring the one conferred on it during construction, and engages the intraocular lens in a stable manner.

According to a preferred embodiment of the invention, the first end (12) of the three-dimensional shape memory implantable element (5) in the memorized configuration is helix-shaped, and this helix has a diameter and a pitch such as to engage the edge of the optical plate (2) between one coil and the other of the helix itself. Preferably, the outside diameter of the propeller is less than 6 mm.

In use, the injector handpiece of the device according to the invention is able not only to bring the shape-memory element located inside it to the operating site, but also to advance its position by means of a mechanism inside the handpiece itself. operated manually by the surgeon. The shape memory element is made to come out of the hollow tubular end of the handpiece near the IOL to be repositioned. For this purpose, the injector handpiece is provided with a system for advancing the three-dimensional shape memory element inside the handpiece itself towards its proximal open end, which system comprises, according to a specific embodiment of the invention, a wheel with an axis orthogonal to the direction of advancement of the shape memory element, partially protruding from the shape of the injector handpiece. The external surface of said wheel is in non-sliding contact with the external surface of a pusher pin which in turn advances the shape memory element housed inside the handpiece.

Materials suitable for making the components of the medical device of the invention other than shape memory elements can be polymeric materials such as polyethylene, polypropylene, hydrophilic or hydrophobic acrylic polymers, nylon, polyurethanes, PVC, PEEK, polyimides, ABS, rigid silicones and elastomers, polybutadiene, silicone fluoride elastomers, metallic materials such as stainless steel, titanium and many others. The choice of the most suitable materials for each of the components of the device is within the reach of any specialized technician in the sector.

According to a preferred embodiment of the present invention, the three-dimensional shape memory implantable element consists of a nickel-titanium alloy wire (Nitinol) with a thickness (or diameter, in the case of a wire circular section) between 0.2 mm and 2 mm, preferably between 0.3 mm and 0.8 mm.

To optimally perform the function of engaging and holding the intraocular lens in place with its first end, the three-dimensional shape-memory implantable element can have a memorized configuration in the first end in the shape of a cylindrical helix (the same as a coil spring). In this case, according to a preferred embodiment of the invention, this first end should consist of at least 1.5 coils, and preferably of a maximum of 2 coils. Any equivalent configuration of the end of the shape memory element which in the pre-set shape is suitable for engaging the IOL at the periphery of the optical plate, holding it in position, is equally possible.

The second end of the three-dimensional shape memory implantable element preferably has a memorized configuration in the shape of a three-dimensional curve which follows the spherical shape of the eyeball in correspondence with the sclera and can be fixed to the scleral wall in the context of a pocket scleral suitably created by the surgeon.

According to a preferred embodiment thereof, the device according to the present invention also comprises an additional fixing means which, sliding along the second end of the shape memory element, allows centering the IOL to be fixed. The additional fixing means can be fixed to the scleral wall, also in this case by means of a pocket arranged by the surgeon in the sclera. In the corresponding embodiment of the invention, the second end of the shape memory implantable element can have a serrated or knurled portion for this purpose, the additional fastening means being coupled with the serrated or knurled portion of said second end.

In particular, the coupling between the second serrated end of the shape memory element and the additional fixing means can be made in a similar way to that of a cable tie, with a stop tooth formed in the through hole of a collar element which can be coupled with said serrated portion of the second end of the shape memory implantable element. Said additional fastening means can be made of soft plastic material, for example silicone.

Two exemplary embodiments of the medical device for scleral fixation according to the invention are detailed below.

With reference to **Figure 2****,** an embodiment of the injector handpiece 4 is schematized through which the shape memory implantable element 5 was released which, by autonomously reacquiring, at the patient's body temperature, the profile given to it during construction, engaged an intraocular lens 1 located in the eye of the patient to be stabilized.

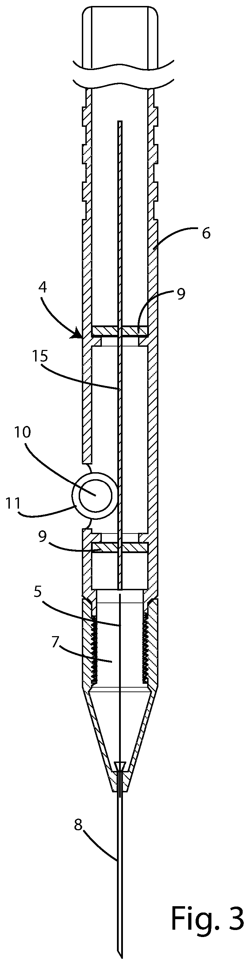

As shown both in Figure 2 and in the longitudinal sectional view of **Figure 3****,** the injector handpiece 4 comprises a hollow tubular main body 6, configured for an ergonomic grip and preferably made of acrylonitrile butadiene styrene (ABS ), a commonly used thermoplastic polymer compound, with an anterior distal section 7 of the same material, from which the cannula 8 protrudes, which can be constituted, for example, by a hollow stainless steel needle of the type for syringes . Preferably, the cannula 8 is of a 25 gauge (25Ga) size, suitable for injecting a filiform shape memory implantable element 5 of the type of nickel-titanium alloy wires (Nitinol).

Again, with reference to Figure 3, inside the injector handpiece 4 the two diaphragms 9 guide the pusher pin 15 which allows the advancement of the shape memory implantable element 5 in sliding towards the cannula 6. This forward sliding is obtained by means of the wheel 10 provided with a non-slip silicone coating 11, rotated by the surgeon's finger. As it advances, the shape memory element 5 comes out of the cannula 8 of the injector handpiece 4 when the handpiece is in the vicinity of the displaced IOL 1 to be engaged. The hollow cannula needle 8 allows access to the operating site in a minimally invasive way.

Once the shape memory implantable element 5 has been released which has engaged the intraocular lens 1, the surgeon can leave the operating site by removing the injector handpiece 4.

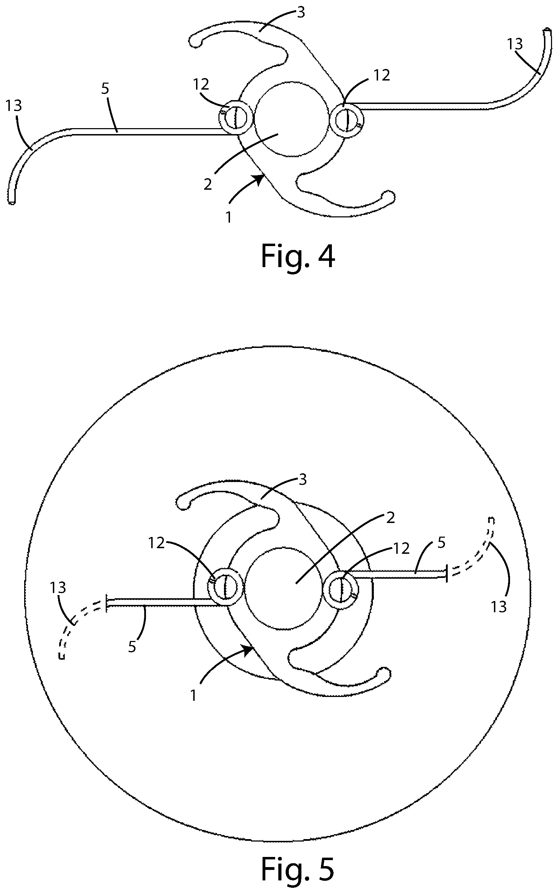

With reference to **Figures 4** and **5****,** a first embodiment of the medical device according to the present invention comprises implantable shape memory elements 5 which in the pre-set configuration have a first end 12 shaped like a cylindrical helix (i.e. like a helical spring) and a second end 13 shaped like a three-dimensional curve which follows the spherical shape of the eyeball in correspondence with the sclera. As shown in Figure 5, the second end 13 of the shape memory implantable element can be fixed in the sclera by providing a suitable scleral pocket, so that its position remains stable and further dislocation events can be avoided.

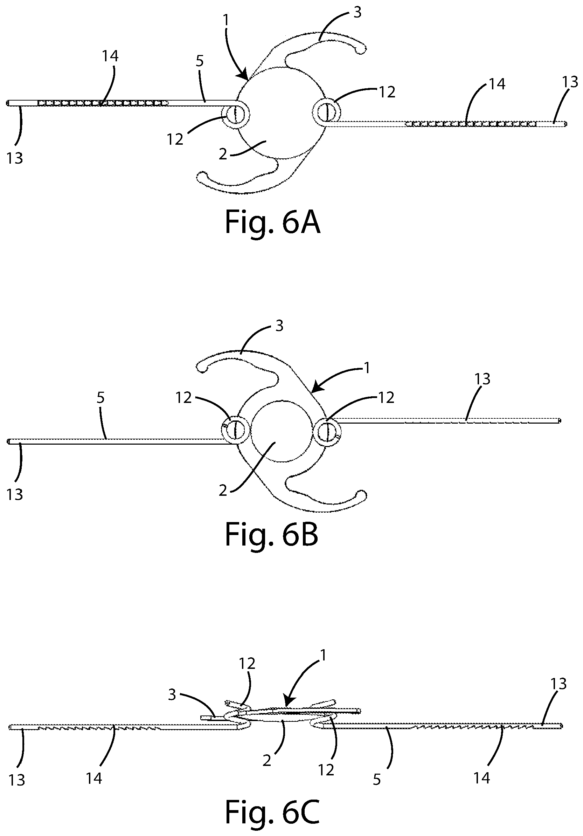

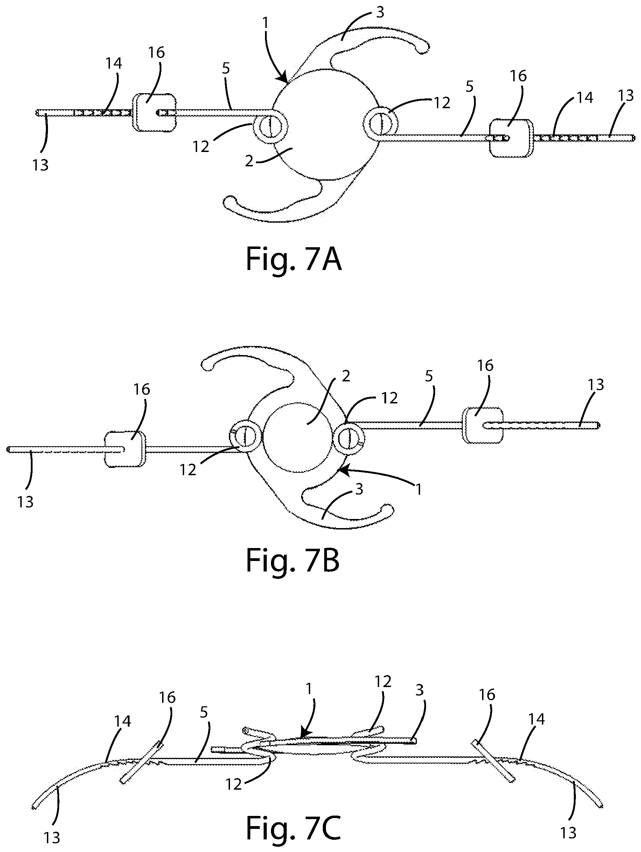

With reference to **Figures 6A, 6B, 6C****,** **7A, 7B** and **7C****,** a second embodiment of the medical device according to the present invention comprises shape memory implantable elements 5 which in the pre-set configuration have a first end 12 shaped like a cylindrical helix as in the previous embodiment, and a second end 13 bearing a serration or knurling 14 intended to couple with an additional fastening means and again shaped like a three-dimensional curve which follows the spherical course of the eyeball corresponding to the sclera (visible only in Figs. 7A, 7B and 7C).

In the Figures of series 6, while the first ends 12 of the shape memory element are represented when they have regained the memorized configuration, the second ends 13, which are the last part of the shape memory element to be placed in contact with the patient's tissues, are shown at a time prior to returning to the memorized configuration.

The Figures of series 7 show the additional element 16 of the fastening means, consisting of a collar or plate of biocompatible material, with a stop tooth formed inside it, which couples with the serrated part of the second end 14 of the shape memory element 5.

In use, once the appropriate seat has been obtained in the scleral wall, the plate 16 of the additional fastening means is made to slide by the surgeon along the shape memory element 5, until the desired centering of the IOL is obtained. Once fully positioned, the surgeon may suture the scleral pocket containing additional fixation, if deemed appropriate. A similar procedure is repeated for the other tie rod, i.e. the shape memory element 5 positioned at 180° from the first.

Preferably, the additional fastening means 15 is made of biocompatible material, and this material is a rigid or soft plastic, rubbery, ceramic or metallic material.

It should be noted that the three-dimensional shape memory elements which can be implanted with the procedure using the device of the present invention can also be more than two and can be placed to hook the intraocular lens in any further position deemed necessary to a stable fixation of the same.

As can be seen from the present description, the aim achieved by the invention is to fix, center and stabilize any type of intraocular lens previously or simultaneously implanted, thus avoiding the removal of an existing intraocular lens in favour of a special lens for fixation and achieving repositioning of the existing lens in a predetermined and safe manner. The medical device of the invention reduces the level of complexity of the scleral fixation technique and the possible damages and complications affecting the patient's visual apparatus.

Furthermore, the use of superelastic shape memory materials for scleral fixation has the fundamental advantage of making this surgical technique extremely minimally invasive, because the shape memory element which acts as a tie rod can be introduced into the seat operation through very small incisions, thus favouring recovery times for the patient.

The present invention has been described with reference to some of its specific embodiments, but it is to be understood that variations or modifications may be made to it by those skilled in the art without thereby departing from the scope of protection defined by the claims.

## Claims

1. . A system for scleral fixation and stabilization of a previously or simultaneously implanted intraocular lens, said intraocular lens having a central optical plate and two or more peripheral elastic haptics, which system comprises:
a medical device aving an injector handpiece (4) with an internal longitudinal cavity ending with a hollow tubular proximal end (7, 8) open towards the outside, suitable for introducing into an operating field of ophthalmic surgery a three-dimensional shape memory implantable element (5) housed therein, having a substantially rectilinear configuration at rest; **characterized in that** it further comprises at least one three-dimensional shape memory implantable element (5) having a substantially rectilinear configuration at rest to be inserted in said injector handpiece (4); wherein said three-dimensional shape memory implantable elements (5) has a memorized configuration capable of engaging and retaining, with a first end (12) in the shape of a helix an intraocular lens (1) at the margins of the optical plate (2) of the same, and with a second end (13) adapted to be anchored to the scleral wall; and wherein said three-dimensional shape memory implantable elements (5) autonomously assumes the memorized configuration when exposed to a predetermined environmental condition corresponding to the conditions inside the eye, with diameter and a pitch of said helix such as to hook the edge of said optical plate (2) between one coil and the other of said helix.

2. **.** The system according to claim 1, in which said predetermined environmental condition is the temperature, and said shape memory element(5) assumes the memorized configuration when brought to the internal temperature of the eye.

3. **.** The system according to each of the preceding claims, wherein said injector handpiece (4) is provided with a system for advancing said three-dimensional shape memory implantable element (5) inside the injector handpiece (4) towards said end proximal tubular open hollow (7, 8), which system comprises a wheel (10) with an axis orthogonal to the direction of advancement of said shape memory element (5), partially protruding from the shape of said injector handpiece ( 4), the outer surface of said wheel (10) being in non-sliding contact with the outer surface of a pusher pin (15) which advances said shape memory element (5) inside the injector handpiece (4).

4. **.** The system according to each of the preceding claims, wherein said shape memory implantable element (5) is a nickel-titanium alloy wire (Nitinol) with a thickness of between 0.2 mm and 2 mm, preferably between 0.3 mm and 0.8 mm.

5. **.** The system according to any one of claims 1 - 3 wherein said shape memory implantable element (5) is a metal alloy wire having said first end (12) with a memorized configuration in the shape of a cylindrical helix, preferably having between 1 .5 and 2 turns.

6. **.** The system according to claim-5, wherein said shape memory implantable element (5) has said second end (13) with a memorized configuration in the shape of a three-dimensional curve which follows the spherical trend of the eyeball in correspondence of the sclera.

7. .The system according to claim 6, wherein said second end (13) of said shape memory implantable element (5) has a serrated (14) or knurled portion.

8. .The system according to claim 7, further comprising a further implantable element, consisting of an additional fixing means (16) which can be coupled with said serrated (14) or knurled portion of said second end (13).

9. **.** The system according to claim 8, wherein said additional fastening means (16) is configured with a stop tooth formed in the through hole of an implantable collar element, which can be coupled and cooperates with said serrated (14) or knurled portion of said second end.

10. .The system according to claims 8 or 9, wherein said additional fixing means (16) is made of biocompatible material, and said material is a rigid or soft plastic, rubbery, ceramic or metallic material.

## Patentansprüche

1. System zur skleralen Fixierung und Stabilisierung einer zuvor oder gleichzeitig implantierten Intraokularlinse, wobei die Intraokularlinse eine zentrale optische Platte und zwei oder mehr periphere elastische Haptiken aufweist, wobei das System Folgendes umfasst: ein medizinisches Gerät, das ein Injektorhandstück (4) mit einem inneren Längshohlraum aufweist, der mit einem hohlen rohrförmigen proximalen Ende (7, 8) endet, das nach außen offen ist und geeignet ist, in ein Operationsgebiet der Augenchirurgie ein darin untergebrachtes dreidimensionales implantierbares Formgedächtniselement (5) einzuführen, das eine im Wesentlichen geradlinige Konfiguration im Ruhezustand aufweist; **dadurch gekennzeichnet, dass** es ferner mindestens ein dreidimensionales implantierbares Formgedächtniselement (5) umfasst, das eine im Wesentlichen geradlinige Konfiguration im Ruhezustand aufweist, um in das Injektorhandstück (4) eingeführt zu werden; wobei dreidimensionale Formgedächtnis-implantierbare Elemente (5) haben eine gespeicherte Konfiguration, die in der Lage ist, mit einem ersten Ende (12) in Form einer Helix oder dreidimensionalen Spirale eine Intraokularlinse (1) an den Rändern der optischen Platte (2) derselben in Eingriff zu nehmen und zu halten, und mit einem zweiten Ende (13), das an der Sklerawand verankert werden kann; und wobei die dreidimensionalen Formgedächtnisimplantierbaren Elemente (5) autonom die gespeicherte Konfiguration einnehmen, wenn sie einer vorbestimmten Umgebungsbedingung ausgesetzt werden, die den Bedingungen im Inneren des Auges entspricht, wobei Durchmesser und eine Steigung der Helix, um den Rand der optischen Platte (2) zwischen einer Spule und der anderen der Helix einzuhaken.

2. System nach Anspruch 1, bei dem die vorbestimmte Umgebungsbedingung die Temperatur ist und das Formgedächtniselement(5) die gespeicherte Konfiguration annimmt, wenn es auf die Innentemperatur des Auges gebracht wird.

3. System nach jedem der vorhergehenden Ansprüche, wobei das Injektorhandstück (4) mit einem System zum Vorschieben des implantierbaren dreidimensionalen Formgedächtniselements (5) innerhalb des Injektorhandstücks (4) in Richtung des proximalen röhrenförmigen offenen Hohlraums (7, 8) versehen ist, wobei das System ein Rad (10) mit einer Achse senkrecht zur Vorschubrichtung des Formgedächtniselements (5) umfasst, das teilweise aus der Form des Injektorhandstücks ( 4) herausragt, wobei die Außenfläche des Rads (10) in nicht gleitendem Kontakt mit der Außenfläche eines Druckstifts (15) steht, der das Formgedächtniselement (5) innerhalb des Injektorhandstücks (4) vorschiebt.

4. System nach einem der vorhergehenden Ansprüche, wobei das implantierbare Formgedächtniselement (5) ein Nickel-Titan-Legierungsdraht (Nitinol) mit einer Dicke zwischen 0,2 mm und 2 mm, vorzugsweise zwischen 0,3 mm und 0,8 mm ist.

5. System nach einem der Ansprüche 1 - 3, wobei das implantierbare Formgedächtniselement (5) ein Metalllegierungsdraht ist, der das erste Ende (12) mit einer gespeicherten Konfiguration in Form einer zylindrischen Spirale aufweist, die vorzugsweise zwischen 1,5 und 2 Windungen aufweist.

6. System nach Anspruch 5, wobei das implantierbare Formgedächtniselement (5) das zweite Ende (13) mit einer gespeicherten Konfiguration in Form einer dreidimensionalen Kurve aufweist, die dem sphärischen Trend des Augapfels entsprechend der Sklera folgt.

7. System nach Anspruch 6, wobei das zweite Ende (13) des implantierbaren Formgedächtniselements (5) einen gezahnten (14) oder gerändelten Abschnitt aufweist.

8. System nach Anspruch 7, ferner umfassend ein weiteres implantierbares Element, bestehend aus einem zusätzlichen Befestigungsmittel (16), das mit dem gezahnten (14) oder gerändelten Abschnitt des zweiten Endes (13) gekoppelt werden kann.

9. System nach Anspruch 8, wobei das zusätzliche Befestigungsmittel (16) mit einem in der Durchgangsbohrung eines implantierbaren Kragenelements ausgebildeten Anschlagzahn ausgebildet ist, der mit dem gezahnten (14) oder gerändelten Abschnitt des zweiten Endes koppelbar ist und zusammenwirkt.

10. System nach Anspruch 8 oder 9, wobei das zusätzliche Befestigungsmittel (16) aus biokompatiblem Material hergestellt ist und das Material ein steifer oder weicher Kunststoff, gummiartiges, keramisches oder metallisches Material ist.

## Revendications

1. . Un système pour la fixation sclérale et la stabilisation d'une lentille intraoculaire implantée précédemment ou simultanément, ladite lentille intraoculaire ayant une plaque optique centrale et deux ou plusieurs haptiques élastiques périphériques, lequel système comprend : un dispositif médical comprenant une pièce à main d'injecteur (4) avec une cavité longitudinale interne se terminant par une extrémité proximale tubulaire creuse (7, 8) ouverte vers l'extérieur, adaptée pour introduire dans un champ opératoire de chirurgie ophtalmique un élément implantable à mémoire de forme tridimensionnel (5) logé dans celui-ci, ayant une configuration sensiblement rectiligne au repos ; **caractérisé en ce qu'**il comprend en outre au moins un élément implantable à mémoire de forme tridimensionnel (5) ayant une configuration sensiblement rectiligne au repos à insérer dans ladite pièce à main d'injecteur (4) ; dans lequel ledit les éléments implantables à mémoire de forme tridimensionnelle (5) ont une configuration mémorisée capable d'engager et de retenir, avec une première extrémité (12) en forme d'hélice ou de spirale tridimensionnelle, une lentille intraoculaire (1) aux marges de la plaque optique (2) de celle-ci, et avec une seconde extrémité (13) adaptée pour être ancrée à la paroi sclérale ; et dans lequel lesdits éléments implantables à mémoire de forme tridimensionnelle (5) adoptent de manière autonome la configuration mémorisée lorsqu'ils sont exposés à une condition environnementale prédéterminée correspondant aux conditions à l'intérieur de l'œil, avec et un pas de ladite hélice de manière à accrocher le bord de ladite plaque optique (2) entre une bobine et l'autre de ladite hélice.

2. **.** Système selon la revendication 1, dans lequel ladite condition environnementale prédéterminée est la température, et ledit élément à mémoire de forme (5) prend la configuration mémorisée lorsqu'il est amené à la température interne de l'œil.

3. **.** Système selon chacune des revendications précédentes, dans lequel ladite pièce à main d'injecteur (4) est pourvue d'un système pour faire avancer ledit élément implantable à mémoire de forme tridimensionnel (5) à l'intérieur de la pièce à main d'injecteur (4) vers ledit creux ouvert tubulaire proximal d'extrémité (7, 8), lequel système comprend une roue (10) avec un axe orthogonal à la direction d'avancement dudit élément à mémoire de forme (5), faisant partiellement saillie de la forme de ladite pièce à main d'injecteur ( 4), la surface extérieure de ladite roue (10) étant en contact non glissant avec la surface extérieure d'une broche de poussée (15) qui fait avancer ledit élément à mémoire de forme (5) à l'intérieur de la pièce à main d'injecteur (4).

4. **.** Le système selon chacune des revendications précédentes, dans lequel ledit élément implantable à mémoire de forme (5) est un fil en alliage nickel-titane (Nitinol) avec une épaisseur comprise entre 0,2 mm et 2 mm, de préférence entre 0,3 mm et 0,8 mm.

5. **.** Système selon l'une quelconque des revendications 1 à 3, dans lequel ledit élément implantable à mémoire de forme (5) est un fil en alliage métallique ayant ladite première extrémité (12) avec une configuration mémorisée en forme d'hélice cylindrique, ayant de préférence entre 1,5 et 2 tours.

6. **.** Système selon la revendication 5, dans lequel ledit élément implantable à mémoire de forme (5) a ladite seconde extrémité (13) avec une configuration mémorisée sous la forme d'une courbe tridimensionnelle qui suit la tendance sphérique du globe oculaire en correspondance avec la sclérotique.

7. **.** Le système selon la revendication 6, dans lequel ladite seconde extrémité (13) dudit élément implantable à mémoire de forme (5) a une partie dentelée (14) ou moletée.

8. **.** Le système selon la revendication 7, comprenant en outre un autre élément implantable, constitué d'un moyen de fixation supplémentaire (16) qui peut être couplé à ladite partie dentelée (14) ou moletée de ladite seconde extrémité (13).

9. **.** Système selon la revendication 8, dans lequel ledit moyen de fixation supplémentaire (16) est configuré avec une dent d'arrêt formée dans le trou traversant d'un élément de collier implantable, qui peut être couplé et coopère avec ladite partie dentelée (14) ou moletée de ladite seconde extrémité.

10. **.** Le système selon les revendications 8 ou 9, dans lequel ledit moyen de fixation supplémentaire (16) est fait d'un matériau biocompatible, et ledit matériau est un matériau plastique rigide ou mou, caoutchouteux, céramique ou métallique.
